# EUROPEAN PATENT APPLICATION

(11) **EP 4 442 218 A1**
(43) Date of publication of application: **09.10.2024**
(21) Application number: 23166919.3
(22) Date of filing: 06.04.2023
(51) Int. Cl.: A61B 34/20, A61B 34/00, A61B 34/10, A61B 17/00, A61B 90/00

(54) **FORS-ENABLED IMAGE LABELLING FOR MACHINE LEARNING MODELS**

(71) Applicant: Koninklijke Philips N.V., 5656 AG Eindhoven (NL)
(72) Inventor: TORJESEN, Alyssa, 5656AG Eindhoven (NL); BYDLON, Torre Michelle, Eindhoven (NL); PAI RAIKAR, Vipul Shrihari, 5656AG Eindhoven (NL); SINHA, Ayushi, 5656AG Eindhoven (NL); LEE, Brian C, 5656AG Eindhoven (NL); RIJHWANI, Damini, 5656AG Eindhoven (NL)
(74) Representative: Philips Intellectual Property & Standards

(57) **Abstract**

An intervention control method for acquiring an image label (78) of an object of interest within an imaging space during an optical shape sensing based interventional procedure. The method involves navigating an interventional device (30) integrated with an optical shape sensor (41) within the imaging space in accordance with the interventional procedure based on a registration of the optical shape sensor (41) to the imaging space. The method further involves deriving an image label (78) of the object of interest within the imaging space from a generation of encoded image data informative of a pose of the object of interest within the imaging space based on a sensing by the optical shape sensor (41) of a navigation of the interventional device (30) within the imaging space in accordance with the interventional procedure.

## Description

### FIELD OF THE INVENTION

The present disclosure generally relates to a generation of training data for machine learning models (e.g., deep neutral networks) utilized for image segmentation, pose estimation, or image registration during an interventional procedure (e.g., minimally-invasive surgery, video-assisted thoracic surgery, vascular procedures, endoluminal procedures, orthopedic procedures), such as, for example, by tracking a position and/or an orientation of an interventional device or anatomical feature relative to a medical imaging system.

### BACKGROUND OF THE INVENTION

Machine learning models, particularly deep neural networks, are used across medical imaging in numerous applications. For example in image guided therapy, deep learning methods have been used to (1) identify interventional devices in a medical image to help guide a procedure, (2) identify a particular anatomical feature in a medical image to be treated, or (3) learn the registration from one medical imaging modality to another medical imaging modality to enable image fusion. These machine learning models are trained on very large imaging datasets, which, in most cases, must be labelled to provide the ground truth for a machine learning model to train on.

In the context of a digital processing of images, image segmentation provides for a detection and a labeling of an object of interest (e.g., an anatomical organ/model or an interventional device) illustrated in a medical image and is implemented for a generation of very large imaging datasets for machine learning models.

### SUMMARY OF THE INVENTION

While machine learning models for image segmentation have proven to be beneficial, there remains a need for improved techniques for generating very large labeled imaging datasets in order to train machine learning models. To this end, the present disclosure utilizes an optical sensor (e.g., an optical (shape) sensor provided by a Fiber Optical RealShape (FORS) fiber) to automate the image labelling process or create large datasets for machine learning models for applications, such as, for example, identification and tracking of interventional devices, identification and tracking of anatomical features, or registration of multiple imaging modalities.

More particular, an optical (e.g. a FORS) sensor uses light along a multicore optical fiber or along an arrangement of a plurality of (single core) optical fibers for device localization and navigation during an interventional procedure. The principle involved makes use of distributed strain measurements in the optical fiber(s) using characteristic Rayleigh backscatter or controlled (Bragg) grating patterns to provide information in the reflected light. The optical detection along the optical fiber begins at a specific point along the optical sensor, known as the launch or z=0, and the subsequent shape, position, strain and/or orientation of the optical sensor are relative to that point. As such, an optical sensor (typically in an optical fiber) can be integrated into an interventional device in order to provide a shape, a position, and/or an orientation of an entirety or a portion of the interventional device also relative to that point, which provides for an optical sensing of interventional device within an image space during an interventional procedure. Optionally the different optical elements of the sensor are arranged to continuously sense shape along the interventional device.

Exemplary embodiments of the present disclosure include, but are not limited to, (1) intervention control systems, (2) image labeling controllers, and (3) an intervention control methods for acquiring an image label of an object of interest within an imaging space.

The image label may be representative of training data for a machine learning model or ground truth data for an imaging process.

The object of interest may be an interventional device or an anatomical object.

The imaging space may be an imaging space delineated by a medical imaging system or a simulated imaging space of a medical imaging system.

Various intervention control systems embodiments of the present disclosure encompass a system for acquiring, during an interventional procedure (e.g., live or simulated), an image label of an object of interest within an imaging space.

The system may employ an interventional device integrated with an optical sensor (or optical sensing system, optionally an optical shape sensing system or sensor) that is operable to be navigated within the imaging space in accordance with the interventional procedure based on a registration of the optical (shape) sensor (or sensing system) to the imaging space.

The system employs an imaging labelling controller configured to derive the image label of the object of interest within the imaging space from a generation of encoded image data informative of a pose of the object of interest within the imaging space based on a sensing by the optical sensor (or optical shape sensor or optical sensing system or optical shape sensing system) of a navigation of the interventional device within the imaging space in accordance with the optical (shape) sensing based interventional procedure.

Various imaging labelling controller embodiments of the present disclosure encompass a controller employing one or more processors to run or execute instructions stored or encoded in a non-transitory machine-readable storage medium, to acquire an image label of an object of interest within an imaging space registered to an optical (shape) sensor integrated with an interventional device navigated within the imaging space in accordance with an interventional procedure (e.g., live or simulated).

The instructions may be arranged to derive the image label of the object of interest within the imaging space from a generation of encoded image data informative of a pose of the object of interest within the imaging space based on a sensing by the optical (shape) sensing system or optical sensor of a navigation of the interventional device within the imaging space in accordance with the interventional procedure.

Various intervention control method embodiments of the present disclosure encompass a method executable by an optional device navigation controller and an image labelling controller for acquiring, during an interventional procedure (e.g., live or simulated), an image label of an object of interest within an imaging space.

The method involves (the optional device navigation controller navigating) an interventional device integrated with an optical (shape)sensor within the imaging space in accordance with the interventional procedure based on a registration of the optical (shape) sensor to the imaging space.

The method further involves an image labelling controller deriving the image label of the object of interest within the imaging space from a generation of encoded image data informative of a pose of the object of interest within the imaging space based on a sensing by the optical (shape) sensing system of a navigation of the interventional device within the imaging space in accordance with the optical (shape) sensing based interventional procedure.

The foregoing embodiments and other embodiments of the present disclosure as well as various structures and advantages of the present disclosure will become further apparent from the following detailed description of various embodiments of the present disclosure read in conjunction with the accompanying drawings. The detailed description and drawings are merely illustrative of the present disclosure rather than limiting, the scope of the present disclosure being defined by the appended claims and equivalents thereof.

### BRIEF DESCRIPTION OF THE DRAWINGS

FIG. 1 illustrates an exemplary embodiment of a medical intervention system in accordance with the present disclosure.
FIG. 2 illustrates an exemplary embodiment of a medical intervention method in accordance with the present disclosure.
FIGS. 3A and 3B illustrate an exemplary embodiment of an optically sensing guidewire and a catheter based medical intervention in accordance with the present invention.
FIGS. 4A and 4B illustrate an exemplary embodiment of a two-dimensional ultrasound based medical intervention in accordance with the present invention.
FIGS. 5A and 5B illustrate an exemplary embodiment of a three-dimensional ultrasound based medical intervention in accordance with the present invention.
FIGS. 6A-6C illustrate an exemplary embodiment of an optically sensing guidewire based medical intervention in accordance with the present invention.
FIG. 7 illustrates a first exemplary embodiment of a scope based medical intervention in accordance with the present invention.
FIG. 8 illustrates a second exemplary embodiment of a scope based medical intervention in accordance with the present invention.
FIG. 9 illustrates an exemplary embodiment of an anatomical feature detection based medical intervention in accordance with the present invention.
FIG. 10 illustrates an exemplary embodiment of an image registration based medical intervention in accordance with the present invention.
FIG. 11 illustrates an exemplary embodiment of a dual imaging modality based medical intervention in accordance with the present invention.
FIG. 12 illustrates an exemplary embodiment of an imaging labelling controller in accordance with the present disclosure.
FIG. 13 illustrates an exemplary embodiment of user interface in accordance with the present disclosure.

### DETAILED DESCRIPTION OF THE PREFERRED EMBODIMENTS

The present disclosure is applicable to numerous and various optical sensing based interventional procedures including, arranged to provide position-, shape-, orientation- and/or position-related information but are not limited to, (1) minimally-invasive procedures utilizing an optical (shape) sensor (e.g., endoscopic hepatectomy, necrosectomy, prostatectomy, etc.), (2) video-assisted thoracic procedures utilizing an optical (shape) sensor (e.g., lobetectomy, etc.), (3) minimally-invasive vascular procedures utilizing an optical (shape) sensor (e.g., via catheters, sheaths, deployment systems, etc.), (4) minimally-invasive medical diagnostic procedures utilizing an optical (shape) sensor (e.g., endoluminal procedures via endoscopes or bronchoscopes), (5) orthopedic procedures utilizing an optical (shape) sensor (e.g., via k-wires, screwdrivers, etc.) and (6) data collection procedures (e.g., benchtop/phantom data, preclinical, etc.).

The present invention is further applicable to a live execution of an optical (shape) sensing based interventional procedure on a patient or a model, and to a simulation of an optical (shape) sensing based interventional procedure on a patient or a model.

The present disclosure improves upon a generation of training data for machine learning models (e.g., deep neutral networks) utilized for image segmentation or pose estimation during an interventional procedure, such as, for example, by tracking a position and/or an orientation of an interventional device or anatomical feature relative to a medical imaging system.

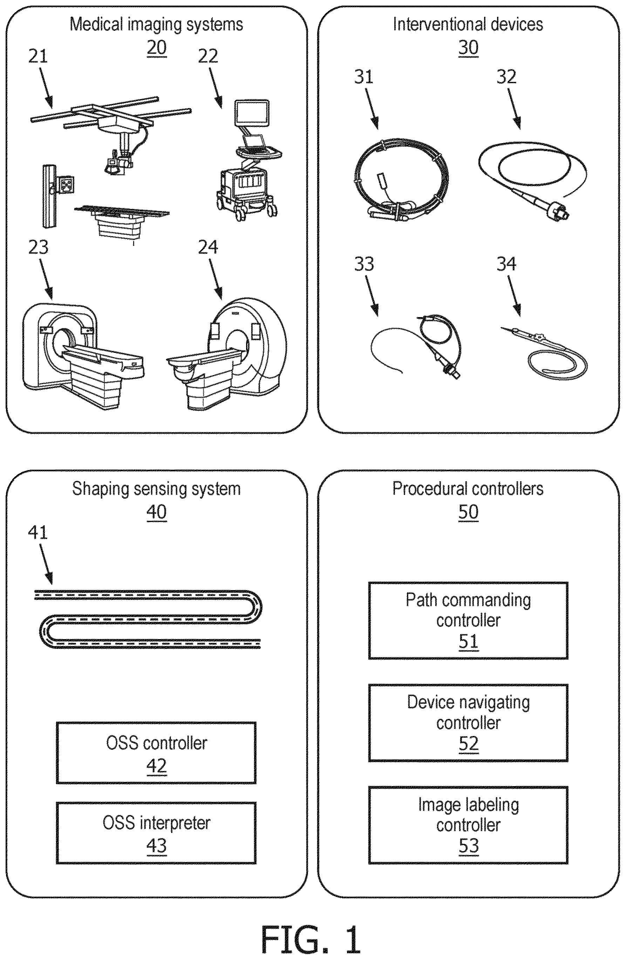

To facilitate an understanding of the present disclosure, the following description of FIG. 1 teaches exemplary embodiments of a medical intervention system in accordance with the present disclosure. From the description of FIG. 1, those having ordinary skill in the art of the present disclosure will appreciate how to apply the present disclosure to make and use additional embodiments of a medical intervention system in accordance with the present disclosure.

Referring to FIG. 1, an exemplary embodiment of a medical intervention system of the present disclosure employs one or more medical imaging systems 20 including, but not limited to, an X-ray imaging system 21, an ultrasound imaging workstation 22, a computed-tomography imaging system 23 and a magnetic resonance imaging system 24. As known in the art of the present disclosure, each of the medical imaging systems 20 are operable to delineate a planar imaging space or a volumetric imaging space for imaging objects within that imaging space.

Still referring to FIG. 1, the exemplary medical intervention system of the present disclosure further employs one or more interventional devices 30 including, but not limited to, a guidewire 31, a catheter 32, a scope 33 and a TEE ultrasound probe 34. As known in the art of the present disclosure, each of the interventional devices 30 provides a surgical function, a therapeutic function and/or a diagnostic function for anatomical objects within a patient or a model during an interventional procedure (live or simulated).

Still referring to FIG. 1, the exemplary medical intervention system of the present disclosure further employs an optical sensing system 40 including an optical sensor 41, an optical sensor controller 42 and an optical sensor interpreter 43.

The optical sensor 41 is arranged to provide position-, orientation-, strain-, shape-related information of data, and the optical sensor interpreter 43 is configured to retrieve this information or data and provide them to a controller for further processing.

In one embodiment, the optical (shape) sensor 41 is a fiber optic Bragg grating based sensor. As known in the art of the present disclosure, a fiber optic Bragg grating (FBG) is a short segment of optical fiber that reflects particular wavelengths of light and transmits all others. This is achieved by adding a periodic variation of the refractive index in the fiber core, which generates a wavelength-specific dielectric mirror. A fiber Bragg grating can therefore be used as an inline optical filter to block certain wavelengths, or as a wavelength-specific reflector.

A fundamental principle behind the operation of a fiber Bragg grating is Fresnel reflection at each of the interfaces where the refractive index is changing. For some wavelengths, the reflected light of the various periods is in phase so that constructive interference exists for reflection and, consequently, destructive interference for transmission. The Bragg wavelength is sensitive to strain as well as to temperature. This means that Bragg gratings can be used as sensing elements in fiber optical sensors. In an FBG sensor, the measurement (e.g., strain) causes a shift in the Bragg wavelength.

One advantage of this technique is that various sensor elements can be distributed over the length of a fiber. Incorporating three or more cores with various sensors (gauges) along the length of a fiber that is embedded in a structure permits a three-dimensional form of such a structure to be precisely determined, typically with better than 1 mm accuracy. Along the length of the fiber, at various positions, a multitude of FBG sensors can be located (e.g. 3 or more fiber sensing cores). From the strain measurement of each FBG, the curvature of the structure can be inferred at that position. From the multitude of measured positions, the total three-dimensional form is determined.

As one of many alternatives to fiber-optic Bragg gratings, the inherent backscatter in conventional optical fiber can be exploited. One such approach is to use Rayleigh scatter in standard single-mode communications fiber. Rayleigh scatter occurs as a result of random fluctuations of the index of refraction in the fiber core. These random fluctuations can be modeled as a Bragg grating with a random variation of amplitude and phase along the grating length. By using this effect in three or more cores running within a single length of multi-core fiber, the 3D shape and dynamics of the surface of interest can be followed.

In operation, as known in the art of the present disclosure, an optical sensing controller 42 (such as an optical shape sensing, or "OSS", controller) controls a generation of sensor data by optical (shape) sensor 41 relative to a reference coordinate system established by or synched with controller 42 whereby the sensor data is communicated to optical sensing (or OSS) interpreter 43 in a data stream of coordinate points associated with the strain sensors of optical (shape) sensor 41.

Also as known in the art of the present disclosure, interpreter 43 interprets the sensor data to ascertain various operational properties of optical (shape) sensor 41 including, but not limited to, a shape of entirety or a portion of optical sensor 41 and a pose (e.g., position and/or orientation) of an entirety or a portion of optical sensor 41 relative to the reference coordinate system.

Still referring to FIG. 1, the exemplary medical intervention system of the present disclosure further employs procedural controllers 50 including, but not limited to, a path commanding controller 51, a device navigating controller 52, and an image labelling controller 53.

In one exemplary embodiment as known in the art of the present disclosure, path commanding controller 51 encompasses a structural configuration (e.g., an application specific main board or an application specific integrated circuit) for autonomously controlling an application of generating commands for navigating an interventional device 20 in accordance with a planned surgical path, a planned therapeutic path and/or a planned diagnostic path within an anatomical region.

In a second exemplary embodiment as known in the art of the present disclosure, path commanding controller 51 encompasses a structural configuration (e.g., an application specific main board or an application specific integrated circuit) for controlling an application of generating commands for navigating an interventional device 20 in accordance a user manipulation of a device coupled to interventional device 20 (e.g., a robot).

In practice, path commanding controller 51 may be installed within or linked to a medical imaging system 20, the OSS system 40 and/or an independent workstation of any type.

Still referring to FIG. 1, as known in the art of the present disclosure, device navigating controller 52 encompasses a structural configuration (e.g., an application specific main board or an application specific integrated circuit) for controlling or managing or monitoring or supervising or processing an application of a manual navigation or an automated (e.g., robotic) navigation of an interventional device 20 integrated with optical (shape) sensor 41 along a planned surgical path, a planned therapeutic path and/or a planned diagnostic path within an anatomical region.

In one exemplary embodiment, device navigating controller 52 employs image-based techniques as known in the art of the present disclosure for facilitating a display of a manual navigation of an interventional device 20 integrated with optical (shape) sensor 41 along a planned surgical path, a planned therapeutic path and/or a planned diagnostic path within an anatomical region.

In a second exemplary embodiment, device navigating controller 52 employs systematic based techniques as known in the art of the present disclosure for controlling a robotic navigation of an interventional device 20 integrated with optical (shape) sensor 41 along a planned surgical path, a planned therapeutic path and/or a planned diagnostic path within an anatomical region.

In practice, device navigating controller 52 may be installed within or linked to a medical imaging system 20, the system 40 and/or an independent workstation of any type.

Still referring to FIG. 1, as will be further described in the present disclosure, image labelling controller 53 encompasses a structural configuration (e.g., an application specific main board or an application specific integrated circuit) for deriving an image label of an object of interest within an imaging space from a generation of encoded image data informative of a pose of the object of interest within the imaging space based on a sensing by the optical (shape) sensor 41 of a navigation of the interventional device 20 within the imaging space in accordance with the interventional procedure. The image label of the object of the interest within the imaging space represents training data for a machine learning or deep learning model. The process of training a deep learning model such as a convolutional neural network (CNN), recurrent neural network (RNN), generative adversarial network (GAN), etc. comprises implementation of iteratively adjusting parameters of the model such that when presented with input data, the model accurately provides the corresponding expected output data. In a particular embodiment, it may be a supervised learning, wherein ground truth labels are used to evaluate the accuracy of the output data generated by the model. During training, the value of a loss function may be computed by comparing the ground truth labels and the output data generated by the model using functions such as mean absolute error, mean squared error, and cross entropy loss, and the loss function is minimized. Training is terminated when the output data generated by the model is within an acceptable range of the ground truth labels, such acceptable range being defined by the user or generated based on predefined parameters of acceptability determined by the user or semi(automatically) generated based on a parametric frame.

In practice, image labelling controller 53 may be installed within or linked to a medical imaging system 20, the system 40 and/or an independent workstation of any type.

Further in practice, two or more of the controllers 51-53 may be integrated within a medical imaging system 20, the system 40 and/or an independent workstation of any type.

Also in practice, device navigating controller 52 and image labelling controller 53 constitute an intervention control system of the present disclosure.

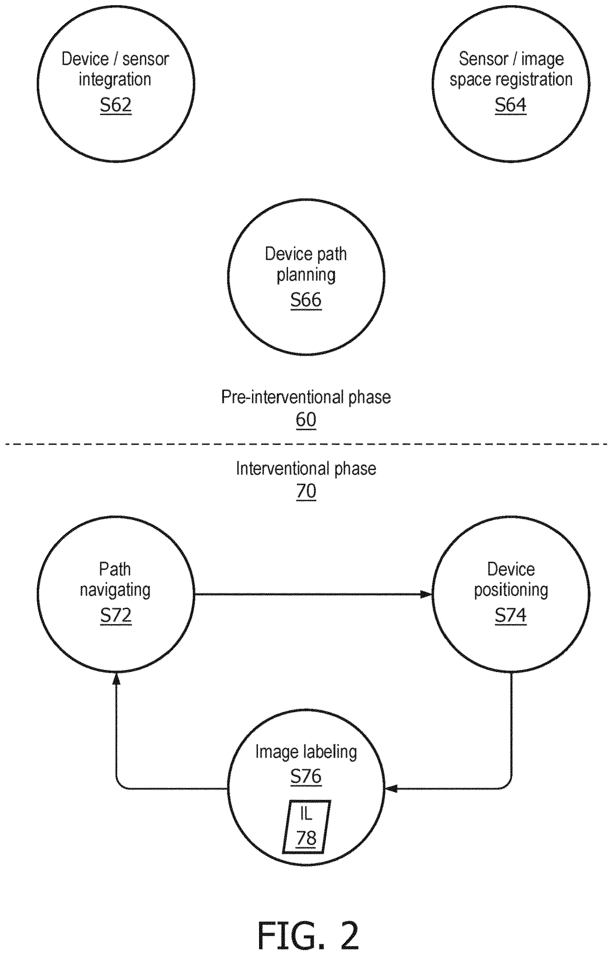

To further facilitate an understanding of the present disclosure, the following description of FIG. 2 teaches exemplary embodiments of a medical intervention method in accordance with the present disclosure. From the description of FIG. 2, those having ordinary skill in the art of the present disclosure will appreciate how to apply the present disclosure to make and use additional embodiments of a medical intervention method in accordance with the present disclosure.

Referring to FIG. 2, an exemplary embodiment of a medical intervention method of the present disclosure encompasses a pre-interventional phase 60 and an interventional phase 70.

Pre-interventional phase 60 implements a device/sensor integration stage S62, a sensor/imaging space registration stage S64 and device path planning stage S66 that can be sequentially and/or concurrently executed in any manner.

In practice, device/sensor integration stage S62 implements techniques as known in the art of the present disclosure for integrating optical (shape) sensor 41 (FIG. 1) with an interventional device 20 (FIG. 1) in a manner suitable for the particular interventional procedure.

In one exemplary embodiment of stage S62, a distal end of optical (shape) sensor 41 is aligned with a distal end of guidewire 21 (FIG. 1) when optical sensor 41 is inserted within guidewire 21 to constitute an optical sensing guidewire (which may be an optical shape sensing guidewire or FORS guidewire).

In a second exemplary embodiment of stage S62, a 3D Hub (Unicath) is attached to an over-the-wire interventional device 20 (e.g., a catheter or an IVUS probe) and a (FORS) guidewire is inserted through the 3D hub and the over-the-wire interventional device 20.

In a third exemplary embodiment of stage S62, for an interventional device 20 that does not go over a (FORS) guidewire, the (FORS) guidewire is rigidly fixed or integrated to the interventional device 20 in a known position. For example, when tracking a (e.g. transesophageal echocardiography "TEE") probe, a cap with a known shape template is retrofitted to the probe and the (FORS) guidewire is integrated into a shape template in the cap. This allows the position and orientation of the probe to be tracked in six (6) degrees of freedom.

Still referring to FIG. 2, in practice, sensor/imaging space registration stage S64 implements techniques as known in the art of the present disclosure for registering optical (shape) sensor 41 with an imaging space delineated by a medical imaging system 20 (FIG. 1) or with a simulated imaging space of a medical imaging system 20, particularly by identifying a tip and a body of the optical sensor 41 in the imaging space and manually aligning a rendering of the optical sensor 41 to match its location in the imaging space.

In one exemplary embodiment of stage S64, a (FORS) guidewire or a (FORS) catheter is registered to x-ray c-arm images by acquiring two images of the (FORS) device from different imaging angles offset by at least thirty (30) degrees. The (FORS) device is identified in each fluoroscopic image whereby a registration to the C-arm isocenter and the device projection onto the imaging plane is calculated. This allows the (FORS) device to be visualized in an imaging space (e.g. fluoroscopy, and/orCT/CBCT space).

In a second exemplary embodiment of stage S64, a (FORS) guidewire or catheter is registered to an ultrasound volume or plane directly by manually identifying a tip and a body of the (FORS) device in an image (e.g. ultrasound) and aligning an optical (shape) sensing (e.g. FORS) rendering of the (FORS) device to match the device location in the image volume. Alternatively, the (FORS) device is registered to an image system (e.g. fluoroscopy or X-ray or CT..), as previously described, then the (ultrasound) probe is registered to this imaging space by a 2D-3D registration method that identifies the probe position and orientation in the image of the imaging system whereby the (FORS) device is visualized in the probe volume in real time given that both modalities are mutually registered to imaging system.

Still referring to FIG. 2, in practice, a device path planning stage S66 might be operated, by e.g. implementing techniques as known in the art of the present disclosure for planning a surgical path, a therapeutic path, and/or a diagnostic path through an anatomical region or a model thereof.

In one exemplary embodiment of stage S66, a path is generated through an image of the anatomical region based on known algorithms with objectives of minimizing the device path length and avoiding the collision with the vital tissue when approaching a target.

Still referring to FIG. 2, interventional phase 70 implements a path commanding stage S72, a device navigation stage S74 and an image labelling stage S76.

In practice, path commanding stage S72 implements techniques as known in the art of the present disclosure for generating navigation commands to navigate the interventional device 20 integrated with the optical (shape) sensor 41 within the registered imaging space delineated by a medical imaging system 20 or with the registered simulated imaging space of a medical imaging system 20.

In practice, device navigation stage S74 implements techniques as known in the art of the present disclosure for executing the navigation commands to thereby navigate the interventional device 20 integrated with the optical (shape) sensor 41 within the registered imaging space delineated by a medical imaging system 20 or with the registered simulated imaging space of a medical imaging system 20. Examples of such techniques include, but are not limited to, a forward predictive model, an inverse predictive model and an imaging predictive model.

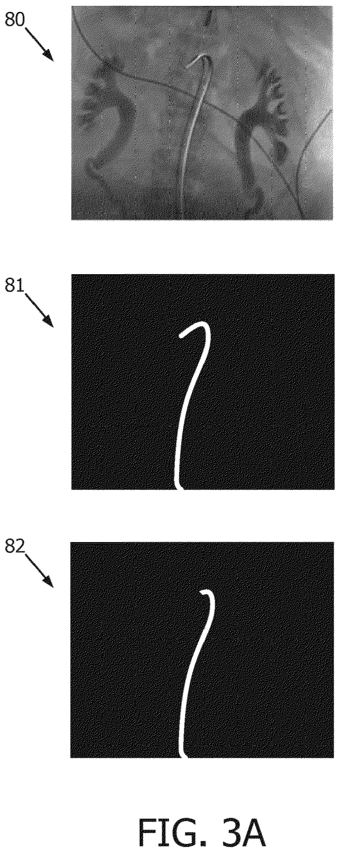

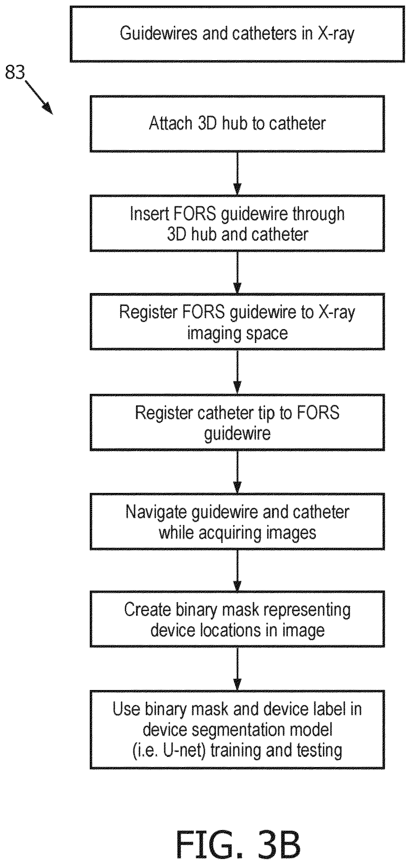

In practice, image labelling stage S76 implements techniques in accordance with the present disclosure for acquiring an image label 78 of an object of interest within an imaging space. As will be further described with the illustrations of FIGS. 3-11, the image labelling of the object of interest within the imaging space is derived from a generation of encoded image data informative of a pose of the object of interest within the imaging space based on a sensing by the optical (shape) sensor 41 of a navigation of the interventional device 20 within the imaging space.

Still referring to FIG. 2, in practice, device navigation stage S74 and image labelling stage S74 constitute an intervention control method of the present disclosure.

Referring to FIG. 3A, in one exemplary embodiment of the medical intervention method of FIG. 2, a rendering of an optically sensing (e.g. a FORS) guidewire and a catheter in the fluoroscopic image 80 is used as a basis for image label 81 for the (FORS) guidewire and an image label 82 for the catheter in fluoroscopy in accordance with a flowchart 83 shown in FIG. 3B.

Specifically, once the (FORS) device is registered to the x-ray space, a projection onto the fluoroscopic image 80 is used directly to derive imaging labels 81 and 82 having a binary encoded pixel data or voxel data defining a shape, a position and an orientation of the (FORS) guidewire or the (FORS) catheter in the fluoroscopic image 80. Imaging labels 81 and 82 are used as the training data for a segmentation network to localize guidewires or catheters in fluoroscopy. The segmentation network may be, as a particular way of implementing it, a convolutional encoder-decoder neural network, such as for example U-Net, V-Net, or SegNet.

Further, an optically sensing (e.g. a FORS) guidewire may be used in combination with a non-optically sensing (e.g. a non-FORS) catheter (or other device or instrument or deployable tool) by attaching the 3D Hub to the catheter to register the catheter to the (FORS) guidewire as previously described herein. This method can be used to detect/label the ground truth position of any over-the-wire device, based on the detected position of the optically sensing guidewire which is registered on one hand to the over-the-wire and on the other hand to the fluoroscopic image (or imaging system which acquires such images). The over-the-wire may be provided with a working channel compatible with the size of a FORS guidewire (inserted in it in a slideable manner) and/or with a luer lock connector at the proximal end. Such over-the-wires may be any navigation/support device (e.g. catheters, sheaths, ...), imaging catheter (e.g. IVUS, OCT), or therapeutic catheter (e.g. atherectomy, angioplasty balloon, aspiration).

If the tip of the (FORS) guidewire is either aligned with the tip of the catheter or extended beyond the tip of the catheter, the visualization of the catheter on fluoroscopy can be used directly as an imaging label. If the guidewire is retracted within the catheter, there will not be any information about the shape of the distal end of the catheter. In that case, a region of interest can be drawn extending from the distal end of the guidewire and an image processing technique as known in the art of the present disclosure (e.g., edge detection and/or nearest neighbor detection) is applied to segment and label the distal end of the catheter.

In practice, if the virtual representation of the (FORS) device has some jitter that could cause small offset errors in the label location, stabilization algorithms as known in the art of the present disclosure may be applied to the (FORS) device to minimize this error. Alternatively, an image label may be created that is wider than the actual device in order to define a region of interest on the fluoroscopic image in which the device may lie. Image processing techniques as known in the art of the present disclosure may then be performed on that region of interest to segment the actual device.

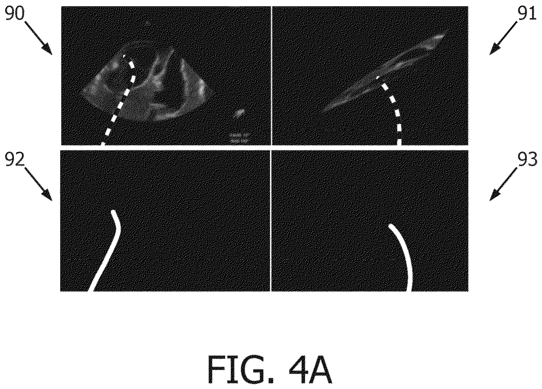

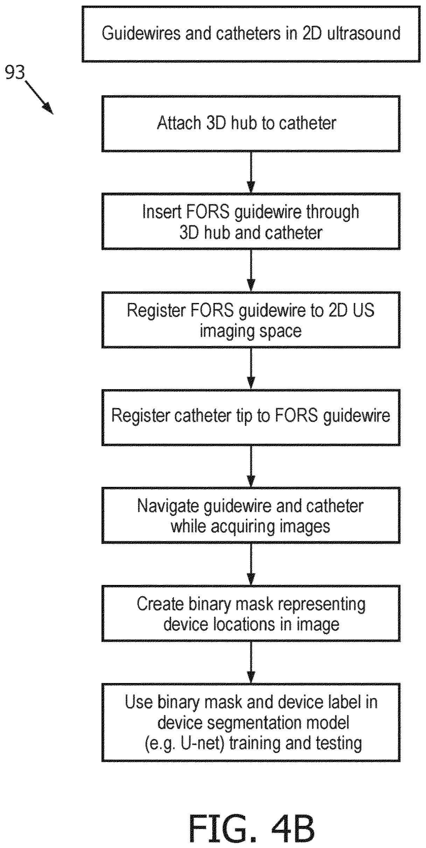

Referring to FIG. 4A, in a second exemplary embodiment of the medical intervention method of FIG. 2, 2D ultrasound images 90 and 91 are used as a basis for image labels 92 and 93 in accordance with a flowchart 94 shown in FIG. 4B. Again, image labels can be obtained from a convolutional neural network, such as for instance U-Net, V-Net or SegNet.

Specifically, an optically sensing (e.g. a FORS) device, or a non-optically sensing (e.g. a non-FORS) catheter used in combination with the 3D Hub is registered to an ultrasound volume as previously described herein. The over-the-wire may be provided with a working channel compatible with the size of a FORS guidewire (inserted in it in a slideable manner) and/or with a luer lock connector at the proximal end. Such over-the-wires may be any navigation/support device (e.g. catheters, sheaths, ...), imaging catheter (e.g. IVUS, OCT), or therapeutic catheter (e.g. atherectomy, angioplasty balloon, aspiration). Once registration is complete, the representations of the (FORS) device in ultrasound images 90 and 91 are used directly as respective imaging labels 92 and 93 to define a shape, a position and an orientation of the (FORS) guidewire or catheter in the 2D ultrasound. The imaging labels 92 and 93 are encoded binary masks that identify which pixels or voxels represent the device in ultrasound images 90 and 91. (FORS) reconstruction occurs at a high frame rate (>50 Hz), therefore the device can be manipulated and labelled in the ultrasound volume in real time.

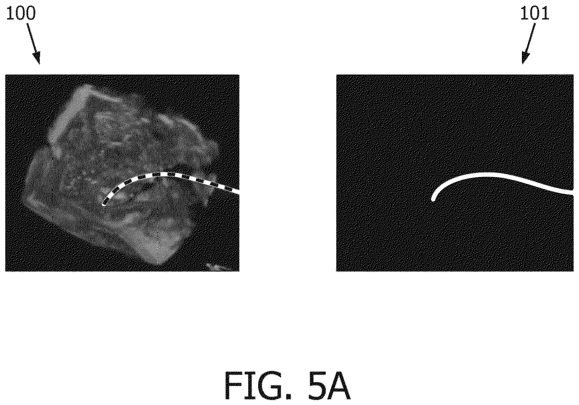

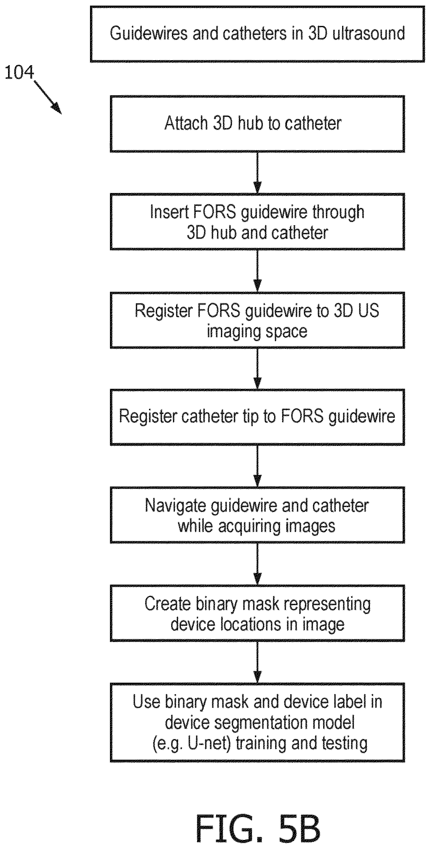

Referring to FIG. 5A, in a third exemplary embodiment of the medical intervention method of FIG. 2, a 3D ultrasound image 100 is used as a basis for an image label 101 in accordance with a flowchart 102 shown in FIG. 5B.

Specifically, an optically sensing (e.g. a FORS) device, or a non-optically sensing (e.g. a non-FORS) catheter used in combination with the 3D Hub is registered to an ultrasound volume as previously described herein. The over-the-wire may be provided with a working channel compatible with the size of a FORS guidewire (inserted in it in a slideable manner) and/or with a luer lock connector at the proximal end. Such over-the-wires may be any navigation/support device (e.g. catheters, sheaths, ...), imaging catheter (e.g. IVUS, OCT), or therapeutic catheter (e.g. atherectomy, angioplasty balloon, aspiration). Once registration is complete, the representations of the (FORS) device in a 3D ultrasound image 100 is used directly as respective imaging label 101 to define a shape, a position and an orientation of the (FORS) guidewire or catheter in the 3D ultrasound. The imaging label 101 is an encoded binary mask that identifies which pixels or voxels represent the device in ultrasound image 100. Again, (FORS) reconstruction occurs at a high frame rate (>50 Hz), therefore the device can be manipulated and labelled in the ultrasound volume in real time.

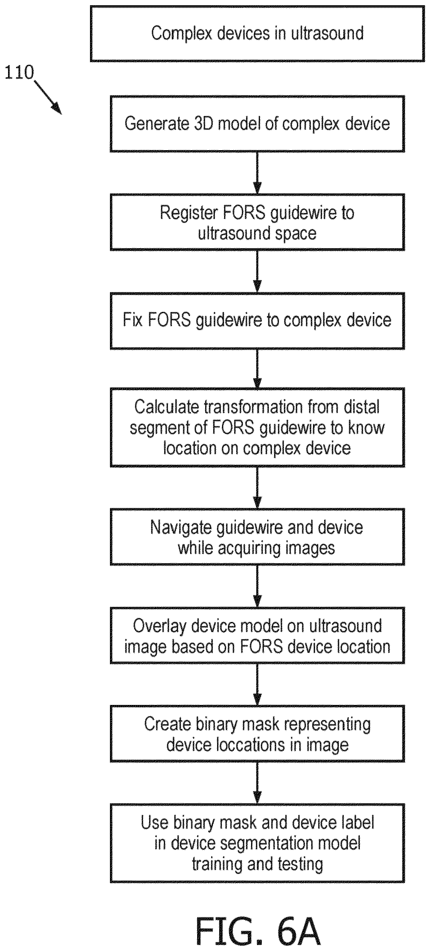

Referring to FIG. 6A, in a fourth exemplary embodiment of the medical intervention method of FIG. 2, an optically sensing (e.g. a FORS) guidewire is fixed to more complex interventional device 20 and tracked in ultrasound to automatically generate device segmentation labels for large image datasets as described in a flowchart 110. Again, segmentation labelling can be obtained from a convolutional neural network, such as for instance U-Net, V-Net or SegNet.

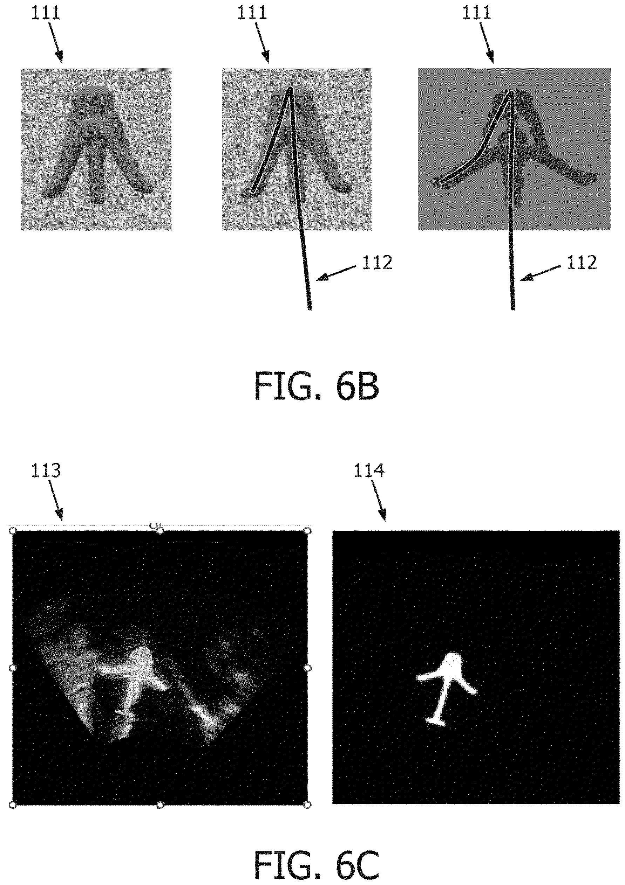

One such interventional device may be the mitral repair device 111a that is deployed under TEE and fluoroscopic guidance. Nevertheless this example should not be limited to such a specific device, this could for instance be any edge-to-edge valve repair device for tricuspid or mitral valves as exemplary applications. A position and an orientation of the mitral repair device 111a can be identified by rigidly fixing an optically sensing (e.g. a FORS) guidewire 112 to the distal end of the deployment system, and along one wing of the clip as shown in FIG. 6B. While this type of tracking would not be feasible when fully deploying the clip in a clinical case, it can be used to generate labeled datasets in phantom and preclinical settings.

A model of the mitral repair device can be positioned in the ultrasound volume 113 at the position and orientation of the mitral repair device as shown in FIG. 6C, which is determined by the (FORS) device. The angle of the wing deployment can also be determined from the (FORS) device because it extends along the wing of the mitral repair device. The model of the mitral repair device can be displayed in real-time in the ultrasound volume, and this model can be used as the image segmentation label 114.

Annotation of such devices and their articulation in ultrasound is challenging as the mechanism of articulation is non-linear due to the tendon driven nature of the system. In this case, optically sensing (e.g. FORS) fiber as illustrated in the images can greatly benefit the annotation process by providing accurate pose information as well as wing state.

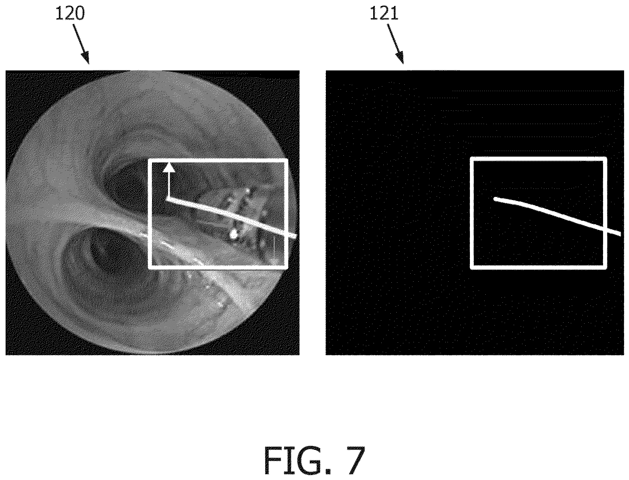

Referring to FIG. 7, in a fifth exemplary embodiment of the medical intervention method of FIG. 2, endoscopes or bronchoscopes generate RGB images of the anatomy and often contain a working channel through which an interventional tool is deployed. It is important to be able to identify the trajectory of the interventional tool in the endoluminal image in order to be able to accurately deliver therapy or take a biopsy. Deep learning models can be created to automatically identify the device and its trajectory in the endoscopic image, which requires a large dataset of labeled images that define the device location in the image.

An optically-sensing (e.g. a FORS) guidewire can be rigidly fixed in the lumen of the interventional tool, or the interventional tool may be placed inside an optically-sensing (e.g. a FORS) catheter, to track the position and orientation of the device. Once the device representation has been registered to the image space, device labels can be automatically generated while the device is manipulated in the field of view of the endoscope. The optically sensing (e.g. the FORS) device in the field of view of the endoscopic image 120 as shown in FIG. 7 can be used directly to create the device label along the optically sensed shape (yellow) affixed to the interventional tool, or a region around the optically sensed shape (yellow) can be defined to create a bounding box label around the relevant portion of the interventional tool in the image. The centerline of the optical sensing device can then be used to train a segmentation process, using e.g. a convolutional neural network (CNN) for image segmentation , such as for example U-Net, V-Net, or SegNet. Said defined bounding box region can then be used to train an object detection network, using e.g. YOLO, or a region-based convolutional neural network (R-CNN).

Similarly, endobronchial ultrasound (EBUS) probes have a side-firing ultrasound array and a working channel through which a biopsy device or other interventional tools may be deployed. The interventional tool can be tracked with an optically-sensing (e.g. a FORS) guidewire, which is registered to the EBUS image, and the angular position of the tool can be detected and labelled. This can then be used as the ground truth training data on which an appropriate model (e.g. using aneural network) is trained to automatically detect the tool and/or the tool trajectory in the EBUS image to provide improved intra-operative guidance, the model may be a model for segmenting the image in order to identify the interventional tool in the image space.

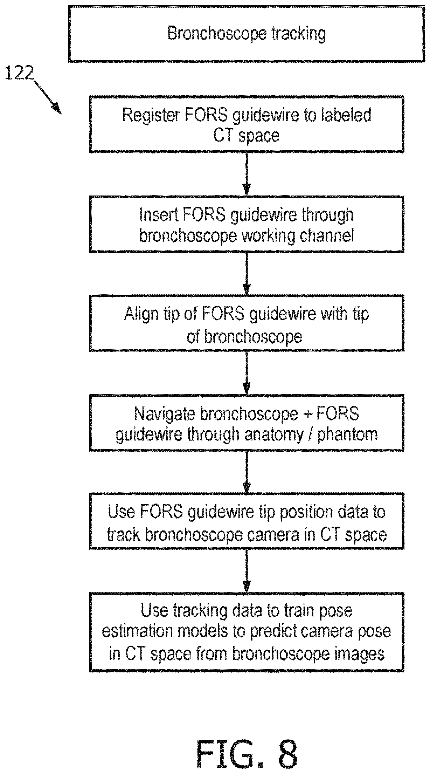

Referring to FIG. 8, in a sixth exemplary embodiment of the medical intervention method of FIG. 2, for bronchoscope (or endoscope, laparoscope, colonoscope, etc.) guided procedures, it is beneficial to be able to track the scope itself as it is navigated through the patient anatomy. For instance, during endobronchial biopsy procedures, bronchoscope tracking can help determine if the physician is navigating towards the target. Several deep learning techniques or other artificial intelligence-based techniques have been explored to learn relative motion between bronchoscope frames through image analysis. That is, by observing how image features change across frames, a network or model can be trained to estimate the relative motion of the bronchoscope camera between those frames. However, obtaining ground truth data for such a training is challenging since typical tracking devices are too big to be attached at the distal end of the scope (i.e., where the camera is located). Instead, trackers (e.g., optical trackers, electromagnetic trackers, etc.) are attached to the handle of the scope. While this may provide accurate albeit offset tracking information for rigid devices like endoscopes and laparoscopes, for flexible devices like bronchoscopes and colonoscopes, the tracking information recorded at the device handle is not reflective of the pose of the device camera.

By fixing an optically sensing (e.g. a FORS) device to the scope, accurate camera tracking information can be obtained by computing the point-to-point (relative) transformation between the (FORS) representations captured at time intervals corresponding to the captured camera frames. Absolute tracking information can also be obtained by acquiring a 3D image (e.g., CBCT) of the scope at the start of the navigation. Using the pose of the scope in the image coordinates as the initial pose and successively adding relative poses from the FORS device to the initial scope pose, the absolute pose of the scope at each frame can be obtained.

The pose parameters extracted with the assistance of the (FORS) device and paired with the corresponding bronchoscope image can be used as ground truth labels for a pose-estimation neural network or model intended to predict the pose of the device based on the appearance of the bronchoscope view as shown in FIG. 8. Similar 3D pose ground truth labels can also be generated for interventional tools inserted into the working channel of the endoscope (see above-mentioned section depicted by FIG. 7) by rigidly fixing an optically-sensing (e.g. a FORS) guidewire to the interventional tool. These labels can be used to train pose-estimation neural networks or other model to predict the pose of the interventional tool based on its appearance in the bronchoscopic field of view. A pose-estimation neural networks may include convolutional neural networks (CNNs) that regress pose parameters (camera position and orientation) from each frame, and/or recurrent neural networks (RNNs) that regress pose parameters using multiple frames.

Another approach is to predict the absolute position and orientation of the endoluminal imaging device relative to some other global coordinate system associated to the patient, such as a 3D model or pre-operative 3D imaging scan. For instance, in order to render the position of an endoluminal device on a preoperative 3D model it is necessary to register the endoluminal image with the preoperative 3D model. Given a registration between preoperative 3D imaging data and intraoperative imaging containing a FORS device (e.g., an intraoperative 3D scan), the FORS device may be affixed or integrated to the endoluminal imaging device to produce absolute position and/or orientation labels associated to the preoperative and/or intraoperative image. A machine learning model could digest images from the endoluminal device and the 3D image with labels and attempt to correlate structures visible in the endoluminal image with the desired label. An exemplary embodiment explaining further such a learning of labels in the context of IVUS is described below.

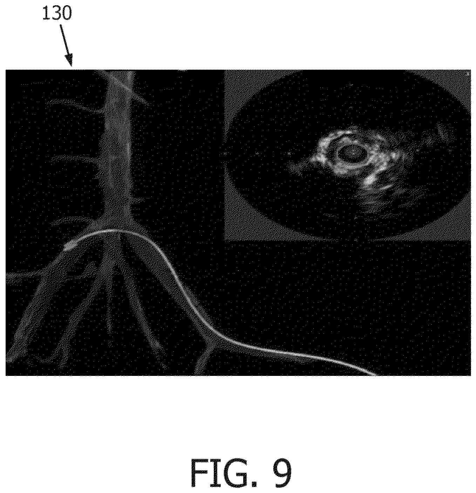

Referring to FIG. 9, in a seventh exemplary embodiment of the medical intervention method of FIG. 2, deep neural networks are often used for automatic detection of anatomical features in 2D and 3D images, such as, for example image 130 shown in FIG. 9. Such automatic detection can be used to for numerous applications, such as optimizing or guiding image acquisition, enhancing visualization of certain features, or guiding a robotic system to automatically maneuver an imaging probe to the optimal imaging view or deliver therapy. Training deep segmentation/detection networks requires a large amount of data in which individual voxels/pixels are assigned a class or bounding boxes are placed around regions of interest, both of which are painstaking to acquire manually. Optical (shape) sensing (e.g. FORS) can be utilized to minimize the need for manual segmentation of large anatomical datasets by instead labelling a single 3D volume then using an optically sensing (e.g. a FORS) device to track an interventional imaging probe that is imaging the pre-segmented volume. If images from two different modalities are registered to each other, labels of anatomical features can be transferred from one imaging modality to the other.

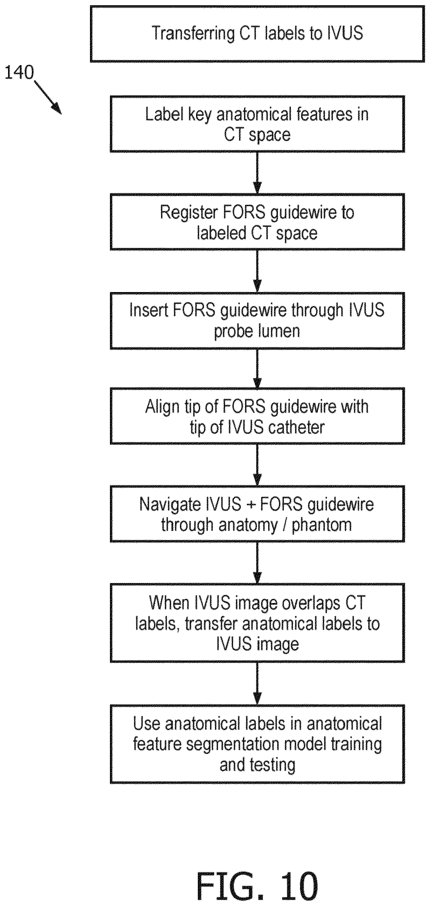

It can be difficult to accurately generate anatomical image labels in an endoluminal image (e.g. an ultrasound image obtained e.g. via an IVUS catheter or an optical image obtained e.g. via an OCT catheter), whereas it is often more straightforward to segment image volumes (obtained e.g. by CT or MR imaging). As an example, if a network is to be trained to automatically identify the aortic lumen and branching vessels during an IVUS pullback, this would typically require ground truth annotations of those anatomical features on each 2D image acquired during the pullback. Instead, this ground truth labelling can be automated by identifying the aortic wall and branching vessels in an image volume. Then, an optically sensing(e.g. a FORS) guidewire is registered to the image volume, the (FORS) guidewire is placed inside the lumen of the IVUS probe with a 3D Hub attached, and the position of the IVUS probe relative to the image volume can be tracked in real-time as shown in FIG. 9. As the IVUS probe is pulled back through the vasculature, the anatomical labels from the image volume can be automatically transferred to the endoluminal (IVUS in this example) image at the corresponding location, as described in the flowchart 140 in FIG. 10. This labelling can then be used to train a convolutional neural network (CNN) or other machine learning model for image segmentation , such as U-Net, V-Net, or SegNet.

A similar approach can also be used to transfer labels to images in other modalities like bronchoscopy. In practice, a post-processing step may be required to compensate for any registration error between the image volume and the probe.

One challenge that can arise when tracking an endoluminal (e.g. IVUS) probe is that optical (shape) sensing (e.g. FORS) can provide 5 degrees of freedom in tracking a coaxial device, such that the rotation along the long axis of the device is unknown. This issue can be mitigated for data collection purposes by creating a small bend in the lumen of the endoluminal probe such that the FORS guidewire can detect this bend and encode the rotation of the endoluminal probe.

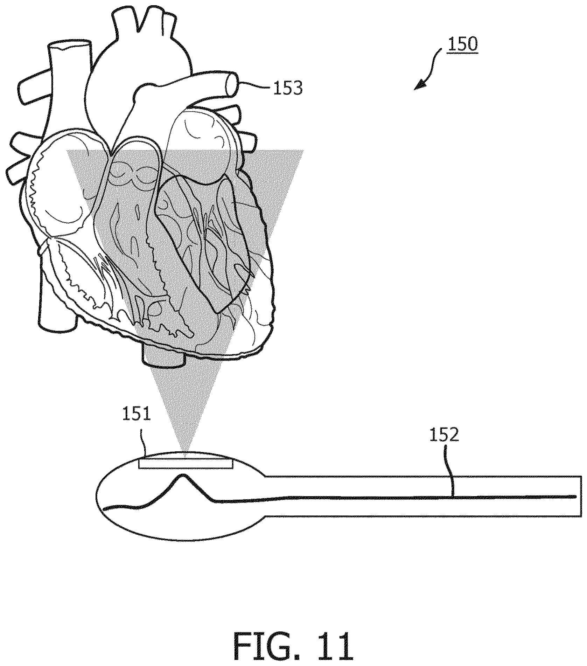

As shown in FIG. 11, anatomical image labels can also be transferred from image volume (e.g. CT or MR imaging) to endoluminal space (via ultrasound or optical catheter) when using external transthoracic echo (TTE) probes or internal TEE probes. If the optically sensing (e.g. FORS) device 152 is registered to the image volume or space 150 and the endoluminal probe 151 (e.g. ultrasound probe) is tracked by rigidly fixing the (FORS) device 152 to the probe, then labels generated in the image volume can be rendered in the probe volume in real-time as the probe is manipulated to view the anatomy from various angles. If imaging cardiac anatomy, cardiac monitoring (e.g. ECG gating) may have to be employed to ensure that image labels that are transferred to the probe space are acquired at the same cardiac phase as the image volume.

In both cases, the optically (shape) sensing (e.g. FORS-) assisted dense template-based segmentation of the probe imagery can be used in place of manually annotated ground truth images for the purpose of training to train a convolutional neural network (CNN) or other machine learning model for image segmentation, such as U-Net, V-Net, or SegNet.. This type of annotation can be produced in mass quantities without manual input.

In an eighth exemplary embodiment of stage S62, it is useful to train a deep neural network or other machine-learning model to automatically register one imaging modality to another to enable image fusion. (Deep) neural networks or other machine-learning model are well-suited to the task of learning non-linear similarity metrics or spatial relationships between different imaging modalities. 2D-3D registration methods can be utilized, where the pose of an interventional imaging probe is identified in an image generated from another imaging system. Alternatively, mutual information can be used to register two 3D volumes to each other that contain the same anatomical features. A pose-estimation neural networks may include convolutional neural networks (CNNs) that regress pose parameters (device position and/or orientation) from each frame, and/or recurrent neural networks (RNNs) that regress pose parameters using multiple frames.

More particularly, deep learning models or other machine-learning model can be trained that use the 2D images to learn the 3D pose of the interventional imaging probe. In order to train such a model, a large set of 2D images visualizing the interventional imaging probe and the known 3D pose of the interventional imaging probe must be used to define the ground truth.

An optically sensing (e.g. a FORS) device can be fixed to a probe (as previously shown in FIG. 11) such as e.g. a TEE or an external ultrasound probe (TTE), to track the probe in 6 degrees of freedom. If the optically sensing (e.g., FORS) device is registered to an imaging system (e.g. x-ray system), then the pose of the probe relative to the imaging system is known. The ground truth transformation matrix between the optically sensing device space and the imaging space along with the sensing data from the optically sensing device corresponding to each image of the probe distal portion (head) can be generated and utilized to provide ground truth pose-image pairs as inputs to the model. Labels may hereby be used to help estimate 3D pose of the probe from 2D images, based on the 2D images of the probe and the corresponding ground truth pose from optical sensing along with transformation matrix so pose is in image space.

An optically sensing (e.g. a FORS) device can also be used to track an over-the-wire imaging probe in 5 degrees of freedom in order to learn the registration between the imaging probe and the image space of an imaging system (e.g. x-ray imaging). If an optically sensing (e.g. a FORS) guidewire is placed inside the lumen of a standard over-the-wire imaging probe, then the rotation about the long axis of the device is unknown. This challenge can be addressed by either modifying the imaging probe such that there is a small bend in the lumen near the distal end such that the (FORS) guidewire can encode the rotation of the probe, or by rigidly fixing or integrating the (FORS) guidewire to a (e.g. an external) surface of the probe in a known orientation.

The pose-image pair automatically generated by the (FORS) device at the time of image acquisition can be used to train a pose-estimation neural network. A pose-estimation neural networks may include convolutional neural networks (CNNs) that regress pose parameters (device position and/or orientation) from each frame, and/or recurrent neural networks (RNNs) that regress pose parameters using multiple frames.

The above embodiment can also improve probe pose estimation which is central to image fusion of ultrasound with x-rays. There are many views where foreshortening of TEE or ICE or other probe or endoscope catheters can cause the pose estimation to fail or return with large errors. In this case, knowing as many degrees of pose as possible (5 DOF with optical sensing fiber (e.g. FORS) can help with registration in challenging views for e.g. trans-gastric views.

In addition to automatic label generation, optically sensing- (e.g. FORS-) assisted imaging can be used to generate synthetic datasets that can be used to train deep learning models in settings where real training data is scarce.

In a ninth exemplary embodiment of stage S62, it could be valuable to display a simulated x-ray image to an interventionalist during a procedure even when the x-ray is not actively being used. Accurate device simulation when generating simulated x-ray images is challenging due to the large parameter space involved in sampling all possible anatomical vs device configurations. Data recorded from an optically (shape) sensing (e.g. a FORS) guidewire could help to constrain the possible configurations that a deep generative model, variational autoencoder and/or diffusion model, or other machine-learning model, would need to learn before being able to simulate accurate imagery, as these would be configurations from previous procedures. One possible model could take as input a desired device configuration (which may be extracted from a last real (x-ray) image) and the background anatomical image (may be the pre-operative CT) and produce a realistic (x-ray) image (DRR) based on these configurations. This model would be trained on image-configuration pairs labelled by a (FORS) device under (x-ray) image.

Synthetic data generation is typically used when it is difficult to obtain actual data. This synthetic data may be then utilized to train deep learning models and is a way to build up a large training dataset. Optical (shape) sensing) (e.g. FORS) may be used to help build a synthetic dataset (for e.g. deep learning). One example of this is in using x-ray images and predicting a future x-ray image. The value of optical (shape) sensing) (e.g. FORS) is that it enables physicians to navigate interventional devices without the use of x-ray. However, there may be procedures where FORS is not used and then physicians must rely on x-ray. It could be advantageous to develop a method to continuously show an "x-ray image" to the physician of what the devices are doing in the body without actually acquiring an x-ray image. In this manner an x-ray image may be acquired but a future frame of the imaging could be predicted. In order to predict future x-ray image frames one must build a training dataset of known x-ray images (with anatomy and device in the image). However, to build this training dataset would require a lot of x-ray images to be acquired. In procedures where optically (shape) sensing (e.g. FORS) is used and the (FORS) devices are registered to x-ray image space, a single x-ray image could be acquired. While navigating the (FORS) device, synthetic images of the x-ray view could be saved by projecting the (FORS) device onto the x-ray image to essentially re-create what the x-ray view would look like. This new synthetic x-ray image dataset could then be used for training purposes.

Optical (shape) sensing (e.g. FORS) could also be used to label data to train a model that provides intraoperative suggestions or transfers knowledge from expert clinicians to less experienced ones. This could be, for example, a network for suggesting the optimal c-arm angle to visualize a particular anatomical feature and the device simultaneously. Training data could be collected while an expert clinician is navigating the FORS device that is registered to the C-arm and CT space. The model would take as inputs the device position and orientation relative to an anatomical feature and predict the C-arm angle of the expert user. Depending on the example, the type of neural network (e.g. CNN) to be chosen could be different. In the case of C-arm angle, pose estimation could be applied. Examples of pose-estimation neural networks include convolutional neural networks (CNNs) that regress pose parameters (C-arm position and/or orientation) from each frame, and/or recurrent neural networks (RNNs) that regress pose parameters using multiple frames.

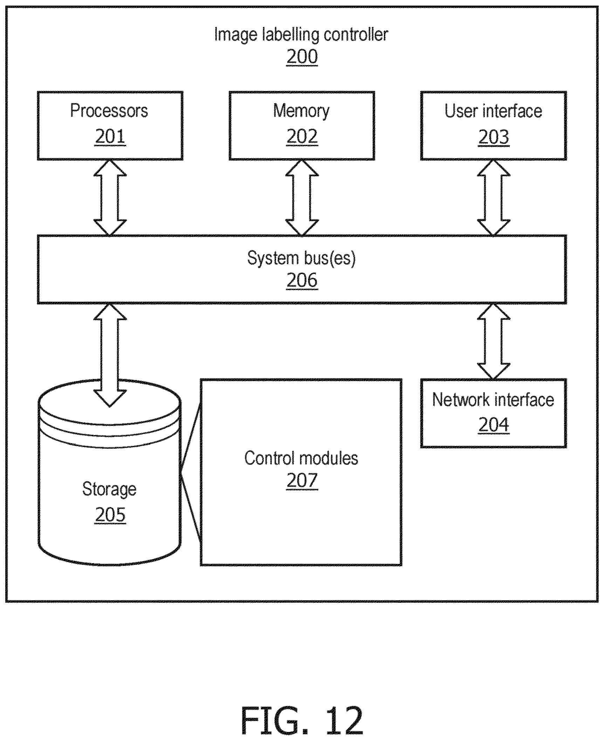

To facilitate a further understanding of the various inventions of the present disclosure, the following description of FIG. 12 teaches an exemplary embodiment of an imaging labelling controller of the present disclosure. From this description, those having ordinary skill in the art will appreciate how to apply various aspects of the present disclosure for making and using additional embodiments of image labelling controller of the present disclosure.

Referring to FIG. 12, an image labelling controller 200 includes one or more processor(s) 201, memory 202, a user interface 203, a network interface 204, and a storage 205 interconnected via one or more system buses 206.

Each processor 201 may be any hardware device, as known in the art of the present disclosure or hereinafter conceived, capable of executing instructions stored in memory 202 or storage or otherwise processing data. In a non-limiting example, the processor(s) 201 may include a microprocessor, field programmable gate array (FPGA), application-specific integrated circuit (ASIC), or other similar devices.

The memory 202 may include various memories, as known in the art of the present disclosure or hereinafter conceived, including, but not limited to, L1, L2, or L3 cache or system memory. In a non-limiting example, the memory 202 may include static random access memory (SRAM), dynamic RAM (DRAM), flash memory, read only memory (ROM), or other similar memory devices.

The user interface 203 may include one or more devices, as known in the art of the present disclosure or hereinafter conceived, for enabling communication with a user such as an administrator. In a non-limiting example, the user interface may include a command line interface or graphical user interface that may be presented to a remote terminal via the network interface 204.

The network interface 204 may include one or more devices, as known in the art of the present disclosure or hereinafter conceived, for enabling communication with other hardware devices. In a non-limiting example, the network interface 204 may include a network interface card (NIC) configured to communicate according to the Ethernet protocol. Additionally, the network interface 204 may implement a TCP/IP stack for communication according to the TCP/IP protocols. Various alternative or additional hardware or configurations for the network interface 204 will be apparent.

The storage 205 may include one or more machine-readable storage media, as known in the art of the present disclosure or hereinafter conceived, including, but not limited to, read-only memory (ROM), random-access memory (RAM), magnetic disk storage media, optical storage media, flash-memory devices, or similar storage media. This storage may be located locally or remotely (e.g. in a cloud databases). In various non-limiting embodiments, the storage 205 may store instructions for execution by the processor(s) 201 or data upon with the processor(s) 201 may operate. For example, the storage 205 may store a base operating system for controlling various basic operations of the hardware. The storage 205 also stores application modules in the form of executable software/firmware for implementing the various functions of the controller 200 as previously described in the present disclosure including, but not limited to, as previously described in the present disclosure.

In practice, controller 200 may be installed within a medical imaging system 20, the OSS system 40 or a stand-alone workstation (e.g., a client workstation or a mobile device like a tablet). Alternatively, components of controller 200 may be distributed medical imaging system 20, the OSS system 40 or the stand-alone workstation. Alternatively components of controller 200 may be located locally and/or remotely (e.g. in a cloud databases).

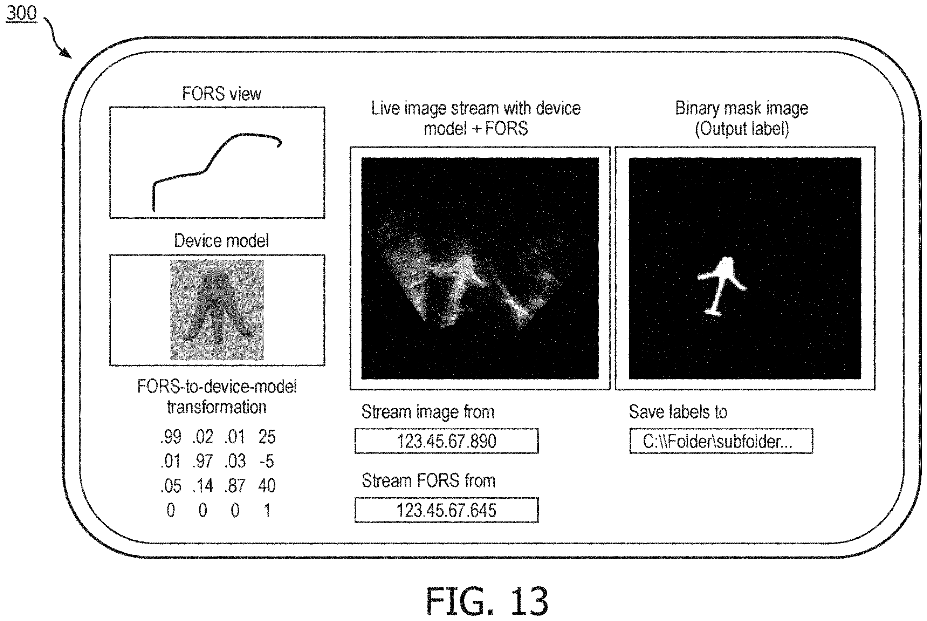

In practice, the present disclosure may provide a user interface for the visualization of an interventional procedure. In one exemplary embodiment of a user interface of the present disclosure as shown in FIG. 13, a user interface 300 may contain various components, such as a way to visualize optical (shape) sensing (e.g. FORS) data / information and imaging data from real-time connections, a way to visualize transformations between various devices or imaging modalities, a way to specify, a way to visualize the image label (mask) being created during acquisition, and a way to specify where to save the image labels.

Referring to FIGS. 1-13, those having ordinary skill in the art of the present disclosure will appreciate numerous benefits of the present disclosure including, but not limited to, the application of the present disclosure to deep learning models developed for numerous interventional procedures in the vascular, cardiac, pulmonary, orthopedic, or other clinical spaces.

The present disclosure has been described with reference to the preferred embodiments. Modifications and alterations may occur to others upon reading and understanding the preceding detailed description. It is intended that the invention be construed as including all such modifications and alterations insofar as they come within the scope of the appended claims or the equivalents thereof.

Further, as one having ordinary skill in the art shall appreciate in view of the teachings provided herein, features, elements, components, etc. disclosed and described in the present disclosure/specification and/or depicted in the appended Figures and/or recited in the Claims can be implemented in various combinations of hardware and software, and provide functions which may be combined in a single element or multiple elements. For example, the functions of the various features, elements, components, etc. shown/illustrated/depicted in the Figures and/or recited in the Claims can be provided through the use of dedicated hardware as well as hardware capable of executing software in association with appropriate software. When provided by a processor, the functions can be provided by a single dedicated processor, by a single shared processor, or by a plurality of individual processors, some of which can be shared and/or multiplexed. Moreover, explicit use of the term "processor" or "controller" should not be construed to refer exclusively to hardware capable of executing software, and can implicitly include, without limitation, digital signal processor ("DSP") hardware, memory (e.g., read only memory ("ROM") for storing software, random access memory ("RAM"), non-volatile storage, etc.) and virtually any means and/or machine (including hardware, software, firmware, combinations thereof, etc.) which is capable of (and/or configurable) to perform and/or control a process.

Moreover, all statements herein reciting principles, aspects, and exemplary embodiments of the present disclosure, as well as specific examples thereof, are intended to encompass both structural and functional equivalents thereof. Additionally, it is intended that such equivalents include both currently known equivalents as well as equivalents developed in the future (e.g., any elements developed that can perform the same or substantially similar functionality, regardless of structure). Thus, for example, it will be appreciated by one having ordinary skill in the art in view of the teachings provided herein that any block diagrams presented herein can represent conceptual views of illustrative system components and/or circuitry embodying the principles of the invention. Similarly, one having ordinary skill in the art should appreciate in view of the teachings provided herein that any flow charts, flow diagrams and the like can represent various processes which can be substantially represented in computer readable storage media and so executed by a computer, processor or other device with processing capabilities, whether or not such computer or processor is explicitly shown.

Having described preferred and exemplary embodiments of the present disclosure, which embodiments are intended to be illustrative and not limiting, it is noted that modifications and variations can be made by persons having ordinary skill in the art in view of the teachings provided herein, including the appended Figures and claims. It is therefore to be understood that changes can be made in/to the preferred and exemplary embodiments of the present disclosure which are within the scope of the present disclosure and exemplary embodiments disclosed, described and taught herein.

Moreover, it is contemplated that corresponding and/or related systems incorporating and/or implementing the device or such as may be used/implemented in a device in accordance with the present disclosure are also contemplated and considered to be within the scope of the present disclosure. Further, corresponding and/or related method for manufacturing and/or using a device and/or system in accordance with the present disclosure are also contemplated and considered to be within the scope of the present disclosure.

## Claims

1. An intervention control system for acquiring an image label (78) of an object of interest within an imaging space during an interventional procedure, the interventional procedure involving an interventional device navigated within the imaging space, which interventional device integrating an optical sensing system arranged to provide an optically-based sensing data, the optical sensing system being registered to the imaging space, the intervention control system comprising:
an imaging labelling controller (53) configured to receive an optically-based sensing data provided from such optical sensing system navigated in accordance with the interventional procedure and to derive the image label (78) of the object of interest within the imaging space from a generation of encoded image data informative of a pose of the object of interest within the imaging space based on the provided optically-based sensing data within the imaging space.

2. The intervention control system of claim 1, wherein the image label (78) is representative training data for a machine learning model or ground truth data for an imaging process.

3. The intervention control system of claim 1,
wherein the object of interest is one of the interventional device (30) or an anatomical object; and
wherein the imaging space is one of an imaging space delineated by a medical imaging system (20) or a simulated imaging space of a medical imaging system (20).

4. The intervention control system of claim 1, wherein the imaging labelling controller (53) is further configured to transfer the image label (78) of the object of interest within the imaging space from an imaging modality associated with the imaging space to a different imaging modality.

5. The intervention control system of claim 1, wherein the imaging labelling controller (53) is further configured to register an imaging modality associated with the image space to a different imaging modality based on the image label (78) of the object of interest within the imaging space.

6. The intervention control system of claim 1, further comprising an interventional device (30) integrated with an optical sensor (41) of the optical sensing system that is operable to be navigated within the imaging space in accordance with the interventional procedure based on a registration of the optical shape sensor (41) to the imaging space and the imaging labelling controller (53) is further configured to implement said derivation of said image label (78) based on a sensing by the optical sensor (41) of the navigation of the interventional device (30) within the imaging space in accordance with the interventional procedure.

7. The intervention control system of claim 6, wherein the optical sensor is an optical shape sensor (41), optionally arranged to continuously sense shape along the interventional device (30).

8. The intervention control system of any of the previous claims, wherein the optically-based data comprises optically-based shape data and/or optically-based position data and/or optically-based orientation data of at least a part of the interventional device.

9. An imaging labelling controller (53), comprising:
at least one processor arranged to execute instructions received from a non-transitory machine-readable storage medium encoded with such instructions, to acquire an image label (78) of an object of interest within an imaging space registered to an optical sensing system integrated with an interventional device (30) navigated within the imaging space in accordance with an interventional procedure;
wherein the executed instructions are arranged to derive the image label (78) of the object of interest within the imaging space from a generation of encoded image data informative of a pose of the object of interest within the imaging space based on the provided optically-based sensing data within the imaging space in accordance with the optical shape sensing based interventional procedure.

10. The imaging labelling controller (53) of claim 9, wherein the image label (78) is representative of training data for a machine learning model or ground truth data for an imaging process,
wherein the object of interest is one of the interventional device (30) or an anatomical object.

11. The imaging labelling controller (53) of claim 9,
wherein the object of interest is one of the interventional device (30) or an anatomical object; and
wherein the imaging space is one of an imaging space delineated by a medical imaging system (20) or a simulated imaging space of a medical imaging system (20).

12. The imaging labelling controller (53) of claim 9, wherein the non-transitory machine-readable storage medium further includes the instructions to transfer the image label (78) of the object of interest within the imaging space from an imaging modality associated with the imaging space to a different imaging modality.

13. The imaging labelling controller (53) of claim 9, wherein the non-transitory machine-readable storage medium further includes the instructions to register an imaging modality associated with the image space to a different imaging modality based on the image label (78) of the object of interest within the imaging space.

14. An intervention control method for acquiring an image label (78) of an object of interest within an imaging space during an interventional procedure, the intervention method comprising:
navigating an interventional device (30) integrated with an optical sensor (41) within the imaging space in accordance with the interventional procedure based on a registration of the optical shape sensor (41) to the imaging space; and
deriving the image label (78) of the object of interest within the imaging space from a generation of encoded image data informative of a pose of the object of interest within the imaging space based on a sensing by the optical sensor (41) of a navigation of the interventional device (30) within the imaging space in accordance with the interventional procedure.
